# FASCICULE DE BREVET EUROPEEN

(11) **EP 0 423 002 B1**
(45) Date de publication et mention de la délivrance du brevet: **14.12.1994**
(21) Numéro de dépôt: 90402775.2
(22) Date de dépôt: 05.10.1990
(51) Int. Cl.: A61K 7/00, A61K 7/32, A61K 9/52

(54) **Composition antimicrobienne pour application sur la peau, applications comme déodorant corporel et bactéricide cutané**
Antimikrobielle Zusammensetzung für die Haut, Verwendung als körperlichem Deodorant und antibakterielles Mittel
Antimicrobial composition for the skin, applied as body deodorant and antibacterial bodyagent

(30) Priorité: 10.10.1989 FR 8913212
(43) Date de publication de la demande: 17.04.1991
(73) Titulaire: CS, F-75008 Paris (FR)
(72) Inventeur: Rase, Didier, F-75016 Paris (FR); Salhi, Ali, F-34980 Saint Gely du Fesc (FR)
(74) Mandataire: Le Guen, Gérard

(56) Documents cités:
- CHEMICAL ABSTRACTS, vol. 112, no. 24, 11 juin 1990, page 414, résumé no. 223263v, Columbus, Ohio, US; & JP-A-01 123 626 (TERUMO CORP.) 16-05-1989
- CHEMICAL ABSTRACTS, vol. 112, no. 2, 8 janvier 1990, page 45, résumé no. 8515y, Columbus, Ohio, US; && JP-A-01 168 337 (TERUMO CORP.) 03-07-1989

## Description

La présente invention concerne des compositions antiseptiques déodorantes qui sont destinées à être appliquées sur la peau, qui possèdent une activité modulable et prolongée et qui contiennent un agent antimicrobien inclus dans des microcapsules, dont la paroi est constituée de collagène et de glycosaminoglycane réticulés.

On sait que la flore cutanée est très variée, aérobie ou anaérobie, composée notamment de staphylocoques epidermidis,aureus et autres microcoques, de corynébactéries aérobies, d'entérobactéries comme Escherichia coli, Klebsiella, Proteus ou de propionobactéries, dans des proportions relatives dépendant de la localisation anatomique, comme mentionné, par exemple, par J. Fleurette dans la Revue du Praticien -30(51) p. 3471-3480 (1980).

On a montré, par ailleurs, que l'odeur de transpiration, en particulier celle des aisselles, était due à l'action de microorganismes sur la sueur, celle-ci ne comportant pas, au moment de la sécrétion, de composés malodorants. On peut se référer par exemple, aux articles de J. J Leyden et coll. dans J. of Investigative Dermatology 77 413-416 (1981), de J.N. Labows dans J. Soc. Cosm. Chem. 33 193-202 (7/1982) ou de Seminars in Dermatology 1 (2) 143-148 qui étudient la nature des microorganismes à l'origine des odeurs désagréables de transpiration; les bactéries Gram - et les propionobactéries ne sont pratiquement pas à l'origine de ces odeurs, tandis que les corynébactéries et autres diphtéroïdes aérobies y sont toujours associés, ainsi qu'à un degré moindre les microcoques.

Aussi, introduit-on maintenant couramment dans les compositions déodorantes corporelles des agents antimicrobiens qui, en inhibant la prolifération bactérienne, suppriment la formation des produits malodorants résultant de la décomposition de la sueur.

L'utilisation d'agents antimicrobiens en dermatologie et en hygiène corporelle est aussi nécessaire.

Par ailleurs, on sait que la microencapsulation des principes actifs pharmaceutiques et cosmétiques dans des membranes à base de polymères hydrosolubles ou biodégradables est fréquemment utilisée pour augmenter la stabilité du produit, masquer une odeur ou un goût, ou encore pour prolonger la durée d'action de la composition.

Les microcapsules, selon l'invention, libèrent leur principe actif essentiellement en présence des microorganismes de la peau qu'il doit combattre. En effet, la paroi des microcapsules est dégradée sous l'action des enzymes protéolytiques sécrétées par la flore cutanée.

Les compositions selon l'invention sont utiles ainsi dans des compositions déodorantes à activité prolongée.

Elles sont, en outre, utiles dans des compositions antiseptiques cutanées à effet rémanent, qui peuvent être appliquées au niveau des plaies superficielles ou des brûlures légères.

La présente invention a ainsi pour objet une composition antimicrobienne pour application sur la peau comprenant des microcapsules contenant un agent antimicrobien caractérisée en ce que la paroi desdites microcapsules est constituée de collagène et de glycosaminoglycane réticulés au moyen d'un agent réticulant.

La paroi des microcapsules est constituée essentiellement de collagène et plus particulièrement d'atélocollagène, ainsi que d'un glycosaminoglycane naturel, comme les chondroïtines sulfates, mucopolysaccharides extraits de la cloison nasale d'ovin, les dermatanes sulfates ou les héparanes sulfates.

L'atélocollagène est un collagène partiellement déréticulé par traitement enzymatique comme décrit dans la demande de brevet FR-A-2 622 104; ce collagène plus soluble ne comporte plus de terminaison télopeptidique.

L'agent réticulant est de préférence un dichlorure d'acide, par exempe le dichlorure de sébacoyle, le dichlorure de téréphtaloyle et le dichlorure d'adipoyle, ou un diisocyanate, par exemple le tolydiisocyanate.

Parmi les agents antimicrobiens couramment utilisés pour assurer l'antiseptie cutanée, on utilisera, de préférence, des composés pratiquement insolubles dans l'eau qui seront introduits dans les microcapsules sous forme d'une solution ou suspension huileuse. Parmi les agents antimicrobiens convenables, on peut citer les carbanilides -comme le cloflucarban, le triclocarban-, les phénols -comme l'hexachlorophéne, le triclosan et les parabens-, les nitrofurannes -comme la nitrofurazone -, les imidazoles -comme le clotrimazole et le miconazole-, ou l'acide undécylénique et ses esters.

L'huile peut être une huile végétale, par exemple l'huile de ricin, un ester d'acide gras, tel qu'un triester de glycérol, par exemple un triglycéride d'acide caprylique et caprique, un diester de glycol, par exemple le dilaurate de propyléneglycol ou encore un ester d'acide gras ou d'alcool gras par exemple le myristate d'isopropyle et l'acétate d'amyle.

Les microcapsules selon l'invention peuvent être préparées par un procédé classique dit de polymérisation interfaciale. La phase huileuse contenant le principe actif et un agent réticulant du collagène soluble dans ce milieu tel que décrit ci-dessus, est versée sous agitation dans la solution aqueuse de collagène et de glycosaminoglycane, en présence d'une base de telle sorte que le pH soit compris entre 7 et 10.

Suivant la concentration dans le milieu de l'agent de réticulation, les parois des microcapsules sont plus ou moins denses et épaisses. On utilise, en général, les chlorures d'acide à raison de 2 g à environ 10 g/100 g de phase huileuse contenant le principe actif; lorsque la concentration est trop faible, les microcapsules sont poreuses; il appartient au spécialiste de déterminer la concentration convenable par quelques essais préliminaires. La concentration du principe actif dans la phase huileuse est fonction de son activité intrinsèque et de sa solubilité; elle est de préférence comprise entre 10% et 60% en poids. Les microcapsules formées sont séparées par centrifugation ou filtration et elles sont lavées à l'eau.

Elles sont conservées de préférence en suspension dans l'eau, éventuellement en présence d'un conservateur lorsqu'elles ne sont pas utilisées rapidement pour la préparation des compositions déodorantes ou antiseptiques selon l'invention.

Les compositions déodorantes selon l'invention, applicables notamment sous les aisselles ou sur les pieds, peuvent être sous forme de bâtons, de gels ou de poudres ou présentées dans des tubes à bille ou des pulvérisateurs; elles contiendront de 0,1 à 2% en poids de microcapsules, selon l'agent antimicrobien utilisé.

Les compositions de l'invention pour l'antiseptie cutanée se présenteront, de préférence, sous forme de crème ou mieux de spray; elles contiendront de 0,5 à 3% en poids de microcapsules.

On peut aussi, pour obtenir un effet immédiat, introduire dans les compositions déodorantes et antiseptiques, une faible quantité d'un agent microbien libre, identique ou non à celui présent dans les microcapsules.

Pour être introduites dans des formulations pour des conditionnements en pulvérisateur sous pression, il est préférable que les microcapsules aient un diamètre de 10 µm, mais elles pourraient avoir jusqu'à 50 µm sans risque de colmater la valve de pulvérisation et dans le cas de gel, crème, bâton et autres, on peut utiliser des microcapsules ayant jusqu'à 100 µm.

Les compositions selon l'invention sont préparées de façon classique, avec les excipients couramment utilisés dans ce domaine. Ces compositions sont essentiellement à base aqueuse pour qu'il n'y ait pas, au cours de la conservation, une extraction progressive du mélange situé à l'intérieur des microcapsules par un solvant de l'huile et/ou de l'agent antimicrobien.

Dans ce qui suit, on décrit quelques exemples de compositions déodorantes selon l'invention ainsi que les résultats obtenus avec ces compositions.

### Préparation de microcapsules et leur étude

### a) Préparation :

On mélange dans un homogénéiseur de type Ultra Turax® tournant à 6000 t/ minute, 40 g de triclosan, et 60 g de triglycérides d'acide caprylique et caprique, vendus par Henkel sous la référence Myritol^{R} 318, qui se présente sous la forme d'une huile neutre de viscosité faible (30 mPa.s à 20°C environ). On ajoute alors 5 ml de chlorure de sébacoyle et on verse le mélange sous agitation dans une solution aqueuse constituée de 4,8 g d'atélocollagène de 1,8 g de chondroïtine-4 sulfate, de 14,4 g de carbonate de sodium (Na₂CO₃) et de 300 ml d'eau; le pH initial de la phase aqueuse est 9,8; l'agitation est réalisée au moyen d'un agitateur planétaire tournant à 600 t/minute; elle est maintenue durant 1 heure avant l'addition d'un même volume d'eau au milieu, où se sont formées les microcapsules. Celles-ci sont séparées du milieu par centrifugation à 2000 t/min.

Après deux lavages par 150 ml d'eau, le dernier culot de centrifugation, qui humide pèse environ 160 g, est mis en suspension dans l'eau à raison de 10g/100 ml, avec éventuellement du Phénonip^{R} à la concentration de 0,5 g/100 ml (essai A); le Phénonip est un conservateur constitué d'une solution à 30% dans le 2-phénoxyéthanol, d'un mélange d'esters méthylique à butylique d'acide p-hydroxybenzoïque.

D'autres essais ont été effectués dans les mêmes conditions, mais avec seulement 2 ml de chlorure d'acide (essai B) ou 3 ml (essai C).

Les microcapsules ainsi préparées ont un diamètre compris entre 1 et 10 µm et contiennent en poids environ 40% de triclosan; elles ne sont pas dégradées en milieu acide, ni par un chauffage à 100°C durant 10 minutes.

### b) Etude de la dégradation des microcapsules

On a mis en contact pendant 24 heures les microcapsules en suspension à 5g/100 ml soit avec une collagénase type II extraite de Clostridium histolyticum, à 37°C et pH 7,6 à la concentration de 1000 U/ml, soit avec une culture de 24 heures de suspension bactérienne, à raison d'1 volume de suspension de microcapsules à 10 g/100 ml pour 9 volumes de culture. Les bactéries étudiées ont été Staphylococcus aureus, Enterobacter cloacae, Serratia marcescens et Proteus vulgaris.

Les microcapsules ont été observées avant et après contact au microscope Leitz Dialux équipé d'un Photoautomat permettant de photographier les vues caractéristiques. On a aussi observé des microcapsules contenant de l'huile, mais pas d'agent antimicrobien, en suspension à 10 g/100 ml dans l'eau contenant 0,5 g/100 ml de conservateur.

Toutes les microcapsules mises en contact avec la collagénase ont éclaté et on n'a observé dans le milieu que des gouttelettes huileuses. L'action des bactéries a été diverse; selon l'état de réticulation de la paroi, et la nature de la bactérie, on a observé un amincissement des parois, leur rupture avec libération du contenu par l'ouverture et déformation de la capsule ou même leur désagrégation; certaines microcapsules n'ont pas été modifiées, mais les germes adhéraient parfois aux parois, formant de petits renflements. L'attaque des parois a été très nette avec E. cloacae et P. vulgaris.

### c) Etude de l'activité antibactérienne

Deux souches bactériennes, E. cloacae et P. vulgaris, ont été mises en contact avec les microcapsules de l'essai A; on a aussi observé un témoin conservateur, c'est-à-dire du Phénonip en solution aqueuse à 0,5% et un témoin composé de l'inoculum bactérien dans un bouillon nutritif. Les résultats de ces essais sont mentionnés dans le tableau I ci-dessous; ils montrent que pour les microcapsules, il y a au bout de 6 heures une réduction de 5 à 6 logarithmes décimaux du nombre initial de bactéries, alors qu'après 24 heures, il n'y a plus aucun germe survivant; ceci traduit bien une libération progressive du principe actif.

**TABLEAU I**

| Souche | Préparation | Nombre de bactéries survivantes aux temps de contact indiqués (h): | | |
|---|---|---|---|---|
| | | 0 | 6 | 24 |
| *Enterobacter cloacae* | Essai A | 8,4 x 10⁸ | 5,7 x 10³ | < 10 |
| | Témoin-conservateur | 1,2 x 10⁹ | 3,7 x 10⁹ | 2 x 10⁹ |
| | Témoin-inoculum | 1,2 x 10⁹ | 3,3 x 10⁹ | 4 x 10⁹ |
| *Proteus* | Essai A | 3,3 x 10⁸ | 4 x 10² | < 10 |
| | Témoin-conservateur | 2,9 x 10⁸ | 2,3 x 10⁹ | 1 x 10¹⁰ |
| | Témoin-inoculum | 2,9 x 10⁸ | 6,5 x 10⁹ | 1,9 x 10¹⁰ |

On donnera ci-après quelques exemples de compositions selon l'invention.

### EXEMPLE 1 :

On prépare un gel bactéricide selon les techniques classiques ayant la composition suivante :

| | |
|---|---|
| carboxypolyméthylène (Carbopol 940) | 0,4 g |
| silicones hydrosolubles | 20 g |
| triclosan | 0,15 g |
| microcapsules de l'essai A | 3 g |
| triéthanolamine | 0,3 g |
| eau | qsp 100 ml |

### EXEMPLE 2 :

On prépare une poudre destinée à être appliquée sur les pieds, comprenant les ingrédients suivants :

| | |
|---|---|
| alcool céto-stéarylique | 2 g |
| octyl-2 dodécanol | 2 g |
| stéarate de zinc | 10 g |
| mono-undécylénate de glycérol | 5 g |
| microcapsules de l'essai A | 3 g |
| piroctone (sel d'éthanolamine) | 0,2 g |
| polyamide-12 (Orgasol^{R} Atochem) | 20 g |
| parfum | 0,2 g |
| talc | qsp 100 g |

### EXEMPLE 3 :

On prépare une suspension ayant la composition suivante qui est introduite dans des flacons munis d'une pompe à pulvérisation :

| | |
|---|---|
| polydiméthylcyclosiloxane (Cyclométhicone)* | 50 g |
| Cyclométhicone et diméthicone* copolyol (Dow Corning) | 25 g |
| Pareth 1,5-3*⁽¹⁾ | 1,5 g |
| triclosan | 0,15 g |
| acide glycyrrhétinique | 1,5 g |
| microcapsules de l'essai A | 3 g |
| parfum | 0,5 g |
| eau | qsp 100 ml |

| | |
|---|---|
| * dénomination CTFA (Cosmetic Toileteries and Fragance Association) | |
| ⁽¹⁾ éther de polyéthylèneglycol et d'alcool gras en C₁₁ à C₁₄ | |

### EXEMPLE 4 :

On prépare une suspension ayant la composition suivante qui est ensuite introduite avec un gaz propulseur dans des bombes de pulvérisation :

| | |
|---|---|
| cocoate de PEG-7 et glycéryle* | 7 g |
| acide glycyrrhétinique | 1,5 g |
| huile de vaseline | 40 g |
| microcapsules de l'essai B | 5 g |
| Cyclométhicone* | 45,5 g |
| parfum | 1 g |

| | |
|---|---|
| * dénomination CTFA (Cosmetic Toileteries and Fragance Association) | |

### EXEMPLE 5 :

On prépare un baton déodorant sans alcool, selon le procédé classique ayant la composition suivante :

| | |
|---|---|
| Cyclométhicone* | 24 g |
| Cyclométhicone* et Dimethicone* copolyol | 24 g |
| Pareth* 15-3 | 1,5 g |
| cire microcristalline d'hydrocarbures ramifiés | 13 g |
| phytostérols | 6 g |
| triclosan | 0,15 g |
| microcapsules de l'essai A | 3 g |
| acid glycyrrhétinique | 1,5 g |
| parfum | 0,2 g |
| H₂O | 26,65 g |

| | |
|---|---|
| * dénomination CTFA (Cosmetic Toileteries and Fragance Association) | |

## Revendications (Revendications pour l'(les) Etat(s) contractant(s) suivant(s): AT, BE, CH, DE, DK, GB, IT, LI, LU, NL, SE)

1. Composition antimicrobienne pour application sur la peau comprenant des microcapsules contenant un agent antimicrobien, caractérisée en ce que la paroi desdites microcapsules est constituée de collagène et de glycosaminoglycane réticulés au moyen d'un agent réticulant.

2. Composition antimicrobienne selon la revendication 1, caractérisée en ce que le collagène est un atélocollagène.

3. Composition antimicrobienne selon l'une des revendications 1 et 2, caractérisée en ce que le glycosaminoglycane est choisi parmi les chondroïtine-4 sulfates, les dermatane-sulfates et les héparane-sulfates.

4. Composition antimicrobienne selon l'une des revendications précédentes, caractérisée en ce que l'agent réticulant est un dichlorure d'acide.

5. Composition antimicrobienne selon la revendication 4, caractérisée en ce que l'agent réticulant est le dichlorure de sébacoyle.

6. Composition antimicrobienne selon l'une des revendications précédentes, caractérisée en ce que les microcapsules contiennent une huile et un agent antimicrobien insoluble dans l'eau.

7. Composition antimicrobienne selon la revendication 6, caractérisée en ce que l'huile est un ester d'acide gras.

8. Composition antimicrobienne selon l'une des revendications précédentes, caractérisée en ce que les microcapsules contiennent du triclosan et un triglycéride d'acide caprylique et caprique.

9. Application d'une composition antimicrobienne selon l'une des revendications précédentes comme déodorant corporel.

10. Composition antiseptique cutanée caractérisée en ce qu'elle comprend une composition antimicrobienne selon l'une des revendications 1 à 8 avec un excipient acceptable pour la peau.

## Revendications (Revendications pour l'(les) Etat(s) contractant(s) suivant(s): ES, GR)

1. Procédé de préparation d'une composition antimicrobienne pour application sur la peau comprenant des microcapsules contenant un agent antimicrobien, caractérisé en ce que l'on mélange des microcapsules dont la paroi est constituée de collagène et de glycosaminoglycane réticulés au moyen d'un agent réticulant, avec au moins un excipient acceptable pour la peau.

2. Procédé selon la revendication 1, caractérisé en ce que le collagène est un atélocollagène.

3. Procédé selon l'une des revendications 1 et 2, caractérisé en ce que le glycosaminoglycane est choisi parmi les chondroïtine-4 sulfates, les dermatanesulfates et les héparane-sulfates.

4. Procédé selon l'une des revendications précédentes, caractérisé en ce que l'agent réticulant est un dichlorure d'acide.

5. Procédé selon la revendication 4, caractérisé en ce que l'agent réticulant est le dichlorure de sébacoyle.

6. Procédé selon l'une des revendications précédentes, caractérisé en ce que les microcapsules contiennent une huile et un agent antimicrobien insoluble dans l'eau.

7. Procédé selon la revendication 6, caractérisé en ce que l'huile est un ester d'acide gras.

8. Procédé selon l'une des revendications précédentes, caractérisé en ce que les microcapsules contiennent du triclosan et un triglycéride d'acide caprylique et caprique.

9. Déodorant corporel comprenant une composition antimicrobienne obtenue selon le procédé de l'une des revendications précédentes.

## Claims (Claims for the following Contracting State(s): AT, BE, CH, DE, DK, GB, IT, LI, LU, NL, SE)

1. Antimicrobial composition for application to the skin, comprising microcapsules containing an antimicrobial agent, characterized in that the wall of the said microcapsules consists of collagen and of glycosaminoglycan which are crosslinked using a crosslinking agent.

2. Antimicrobial composition according to Claim 1, characterized in that the collagen is an atelocollagen.

3. Antimicrobial composition according to either of Claims 1 and 2, characterized in that the glycosaminoglycan is chosen from 4-chondroitin sulphates, dermatan sulphates and heparan sulphates.

4. Antimicrobial composition according to one of the preceding claims, characterized in that the crosslinking agent is a diacyl chloride.

5. Antimicrobial composition according to Claim 4, characterized in that the crosslinking agent is sebacoyl dichloride.

6. Antimicrobial composition according to one of the preceding claims, characterized in that the microcapsules contain an oil and a water-insoluble antimicrobial agent.

7. Antimicrobial composition according to Claim 6, characterized in that the oil is a fatty acid ester.

8. Antimicrobial composition according to one of the preceding claims, characterized in that the microcapsules contain triclosan and a caprylic and capric acid triglyceride.

9. Application of an antimicrobial composition according to one of the preceding claims as a body deodorant.

10. Antiseptic skin composition, characterized in that it comprises an antimicrobial composition according to one of Claims 1 to 8 with an excipient which is suitable for the skin.

## Claims (Claims for the following Contracting State(s): ES, GR)

1. Process for the preparation of an antimicrobial composition for application to the skin comprising microcapsules containing an antimicrobial agent, characterized in that microcapsules, the wall of which consists of collagen and of glycosaminoglycan which are crosslinked using a crosslinking agent, are mixed with at least one excipient which is suitable for the skin.

2. Process according to Claim 1, characterized in that the collagen is an atelocollagen.

3. Process according to either of Claims 1 and 2, characterized in that the glycosaminoglycan is chosen from 4-chondroitin sulphates, dermatan sulphates and heparan sulphates.

4. Process according to one of the preceding claims, characterized in that the crosslinking agent is a diacyl chloride.

5. Process according to Claim 4, characterized in that the crosslinking agent is sebacoyl dichloride.

6. Process according to one of the preceding claims, characterized in that the microcapsules contain an oil and a water-insoluble antimicrobial agent.

7. Process according to Claim 6, characterized in that the oil is a fatty acid ester.

8. Process according to one of the preceding claims, characterized in that the microcapsules contain triclosan and a caprylic and capric acid triglyceride.

9. Body deodorant comprising an antimicrobial composition which is obtained according to the process of one of the preceding claims.

## Patentansprüche (Patentansprüche für folgende(n) Vertragsstaat(en): AT, BE, CH, DE, DK, GB, IT, LI, LU, NL, SE)

1. Antimikrobielle Zusammensetzung für den Auftrag auf die Haut, die ein Antimikroben-Mittel enthaltende Mikrokapseln enthält, dadurch gekennzeichnet, daß die Wand der genannten Mikrokapseln aus Kollagen und Glycosaminoglycan besteht, die mit einem Vernetzungsmittel vernetzt sind.

2. Antimikrobielle Zusammensetzung nach Anspruch 1, dadurch gekennzeichnet, daß es sich bei dem Kollagen um ein Atelokollagen handelt.

3. Antimikrobielle Zusammensetzung nach einem der Ansprüche 1 und 2, dadurch gekennzeichnet, daß das Glycosaminoglycan ausgewählt wird aus den 4-Chondroitin-sulfaten, den Dermatansulfaten und den Heparansulfaten.

4. Antimikrobielle Zusammensetzung nach einem der vorhergehenden Ansprüche, dadurch gekennzeichnet, daß das Vernetzungsmittel ein Säuredichlorid ist.

5. Antimikrobielle Zusammensetzung nach Anspruch 4, dadurch gekennzeichnet, daß das Vernetzungsmittel das Sebacoyldichlorid ist.

6. Antimikrobielle Zusammensetzung nach einem der vorhergehenden Ansprüche, dadurch gekennzeichnet, daß die Mikrokapseln ein Öl und ein in Wasser unlösliches Antimikroben-Agens enthalten.

7. Antimikrobielle Zusammensetzung nach Anspruch 6, dadurch gekennzeichnet, daß es sich bei dem Öl um einen Fettsäureester handelt.

8. Antimikrobielle Zusammensetzung nach einem der vorhergehenden Ansprüche, dadurch gekennzeichnet, daß die Mikrokapseln Triclosan und ein Caprylsäure- und Caprinsäuretriglycerid enthalten.

9. Verwendung der antimikrobiellen Zusammensetzung nach einem der vorhergehenden Ansprüche als Körper-Desodorant.

10. Antiseptische Haut-Zusammensetzung, dadurch gekennzeichnet, daß sie eine antimikrobielle Zusammensetzung nach einem der Ansprüche 1 bis 8 mit einem für die Haut akzeptablen Exzipienten enthält.

## Patentansprüche (Patentansprüche für folgende(n) Vertragsstaat(en): ES, GR)

1. Verfahren zur Herstellung einer antimikrobiellen Zusammensetzung für den Auftrag auf die Haut, die ein Antimikroben-Agens enthaltende Mikrokapseln enthält, dadurch gekennzeichnet, daß man die Mikrokapseln, deren Wand aus Kollagen und Glycosaminoglycan besteht, die mit einem Vernetzungsmittel vernetzt sind, mit mindestens einem für die Haut akzeptablen Exzipienten mischt.

2. Verfahren nach Anspruch 1, dadurch gekennzeichnet, daß es sich bei dem Kollagen um ein Atelokollagen handelt.

3. Verfahren nach einem der Ansprüche 1 und 2, dadurch gekennzeichnet, daß das Glycosaminoglycan ausgewählt wird aus den 4-Chondroitin-sulfaten, den Dermatansulfaten und den Heparansulfaten.

4. Verfahren nach einem der vorhergehenden Ansprüche, dadurch gekennzeichnet, daß es sich bei dem Vernetzungsmittel um ein Säuredichlorid handelt.

5. Verfahren nach Anspruch 4, dadurch gekennzeichnet, daß es sich bei dem Vernetzungsmittel um das Sebacoyldichlorid handelt.

6. Verfahren nach einem der vorhergehenden Ansprüche, dadurch gekennzeichnet, daß die Mikrokapseln ein Öl und ein in Wasser unlösliches Antimikroben-Agens enthalten.

7. Verfahren nach Anspruch 6, dadurch gekennzeichnet, daß es sich bei dem Öl um einen Fettsäureester handelt.

8. Verfahren nach einem der vorhergehenden Ansprüche, dadurch gekennzeichnet, daß die Mikrokapseln Triclosan und ein Caprylsäure- und Caprinsäuretriglycerid enthalten.

9. Körper-Desodorant, das eine antimikrobielle Zusammensetzung enthält, wie sie nach dem Verfahren nach einem der vorhergehenden Ansprüche erhalten wurde.
